# EUROPEAN PATENT SPECIFICATION

(11) **EP 2 589 494 B1**
(45) Date of publication and mention of the grant of the patent: **26.09.2018**
(21) Application number: 11800876.2
(22) Date of filing: 29.06.2011
(51) Int. Cl.: B41F 13/56, B41F 33/00, B41F 33/14, B31B 50/88, B31B 50/00

(54) **CARTON FORMER, INSPECTION DEVICE, AND PRINT REGISTER CONTROL METHOD FOR CARTON FORMER**
KARTONFORMVORRICHTUNG, INSPEKTIONSVORRICHTUNG UND DRUCKREGISTERKONTROLLVERFAHREN FÜR DIE KARTONFORMVORRICHTUNG
CARTONNEUSE, DISPOSITIF DE CONTRÔLE ET PROCÉDÉ DE COMMANDE D'ALIGNEMENT D'IMPRESSION POUR UNE CARTONNEUSE

(30) Priority: 29.06.2010 JP 2010148179
(43) Date of publication of application: 08.05.2013
(73) Proprietor: Mitsubishi Heavy Industries Machinery Systems, Ltd., Hyogo-ku Kobe-shi (JP); Futec Inc., Kagawa 761-0301 (JP)
(72) Inventor: SUZUKI, Yasunari, Mihara-shi Hiroshima 729-0393 (JP); IORI, Shinya, Mihara-shi Hiroshima 729-0393 (JP); KOJIMA, Tooru, Takamatsu-shi Kagawa 761-0301 (JP)
(74) Representative: Intès, Didier Gérard André
(86) International application number: PCT/JP2011/064870
(87) International publication number: WO 2012/002414

(56) References cited:
- EP-A1- 0 598 490
- WO-A1-2008/099195
- JP-A- 11 039 492
- JP-A- 2006 130 795
- JP-A- 2007 062 374
- JP-A- 2009 078 462
- JP-A- 2010 052 211
- US-A- 4 604 083

## Description

### TECHNICAL FIELD

The present invention relates to a box producing apparatus that prints multiple colors on sheet faces, and particularly to a box producing apparatus and an inspection unit provided with units to correct registers (adjust registers) for the print colors, and a print register control method for a box producing apparatus.

### BACKGROUND ART

Some box producing apparatuses that produce boxes from box material sheets (e.g., corrugated board sheets) include a feed section, a printing section, a slotter creaser section, a die cutting section, a folding section (folder gluer section), and a counter ejector section, in this order, from the upstream side. In such a box producing apparatus, a feed section is loaded with box material sheets, which are fed on a sheet by sheet basis, to a printing section, where multiple print units provided therein print respective colors. Then, slotting and scoring are performed by a slitter and a scorer in a slotter creaser section, and cutting is performed in a die cutting section. Then, in a folding section, after gluing is performed by a glue gun, folding and bonding are performed, and the resultant sheets are counted by a counter ejector section. The sheets are stacked into a stack of a certain number, and are transferred to the subsequent step. Note that other box producing apparatuses include no folding section, and sheets are cut in a die cutting section without being glued, folded, or bonded and the cut sheets are counted in a counter ejector section, followed by stacking to a certain number and a transferring to the subsequent step.

A technique has been developed for such a box producing apparatus, wherein a camera is provided in a main part in the box producing apparatus to capture an image of a pass sheet as a master image in advance. In operation of the box producing apparatus, an image of a sheet that went through each of the processes is captured by the camera, and the image of the sheet captured by the camera during the operation is compared against the master image to determine failures by inspecting the qualities of the print position, missing color, and the gluing position. For example, a print failure inspection unit is described in Patent Reference 1. Further, another technique has been developed wherein an optoelectronic sensor, rotary encoder, or the like is provided in a main part in the box producing apparatus to determine failures in slotting lengths or die cutting positions after processing.

In order to improve the merchantability of produced corrugated board boxes, proper shaping of the boxes and proper processing of cut surfaces and the like are essential. To achieve these goals, slotting positions set by a slotter in a slotter creaser section, gluing positions set by a glue gun for gluing in a folding section, and the like are required to be precisely set. Further, precise positioning of printed pictures and letters (hereinafter, they are collectively referred to as pictures) is quite essential for improving the merchantability of corrugated board boxes.

Apparatuses that inspect product failures as described above, however, are only capable of removing failed articles from products for shipping good products only, but cannot prevent the failed articles from being produced.

Hence, in general, elements in a box producing apparatus are adjusted in advance by feeding a single test sheet. Then, an operator inspects the print positions and the like on the sheet, and makes the following five pre-adjustment by means of button operations:
(1) Adjustment of the index position (position in the print vertical direction) of each color print unit in a printing section;
(2) Adjustment of the position in the print width direction of each color print unit in the printing section;
(3) Adjustment of the index position for a slotter;
(4) Adjustment of the index position for a die cutting; and
(5) Adjustment of the gluing position by a glue gun.

### PRIOR ART REFERENCE

### PATENT DOCUMENT

Patent Reference 1: Japanese Laid-open Patent Publication No. 2001-315309
US4604083 discloses a machine for manufacturing folded boxes, comprising: a printing section comprising a plurality of print units: which determines an amount of register displacement in each of the print units, controls a register adjustment mechanism provided in each of the print units based on the amount of register displacement to correct a register of the print. EP0598490 discloses a colour registration system or a printing press and WO 2008/099195 discloses a method of manufacturing blanks for packaging.

### DISCLOSURE OF THE INVENTION

### Problems to be Solved by the Invention

During a pre-adjustment for elements in a box producing apparatus as described above, the results of processing of a test-fed sheet is compared against a sample (reference), a deviation (amount of displacement) of the process result with respect to the sample is determined, and the positions of color print units in a printing section, a slotter, a die cutter, and a glue gun (print position, slotting position, cutting position, and gluing position) are adjusted based on the deviation.

After adjusting the positions of the elements, the positions (print position, slotting position, cutting position, and gluing position) are corrected according to the adjustments. Correcting the positions based on the amounts of displacements requires skill of an operator, and particularly making fine-adjustment for a great deviation to the sample is difficult even for a skillful operator, and multiple test feeds are required to match the test result to the sample.

Once a pre-adjustment is made, the results are stored for each order. By storing the results, the deviation between a test-fed sheet and a sample can be reduced when the stored adjustment values are preset for that order. Thus, a pre-adjustment can be completed after a single test feed. In the case of a new order, however, since an initial deviation is significant, multiple test feeds are required to be performed, which are burdensome to an operator and longer time period is required for the adjustment.

The present invention is made in light of the aforementioned issues, and an object thereof is to provide a box producing apparatus, an inspection unit, and a print register control method for a box producing apparatus that facilitate a pre-adjustment of the box producing apparatus, reduce the burden on an operator, and can manufacture boxes in a suitable and precise manner.

### MEANS TO SOLVE PROBLEMS

In order to achieve the above-identified object, a box producing apparatus of the present invention is a box producing apparatus as defined by claim 1 and comprises: a printing section comprising a plurality of print units; an image obtaining unit that obtains an image of a printed face which is printed on a box material sheet by the printing section; and a register correction control unit that compares a specific picture in a master image (master pattern) of a sample for the print, and the specific picture in the image of the printed face obtained by the image obtaining unit to determine an amount of register displacement in each of the print units, and controls a register adjustment mechanism provided in each of the print units based on the amount of register displacement to correct a register of the print.

Preferably, the register correction control unit detects the specific picture in a region of the printed face where the specific picture is specified to exist in advance based on the master image, and compares the specific picture detected in the printed image against a specific picture in the master image.

The specific picture is a picture including at least an area where a plurality of colors do not overlap, and the register correction control unit comprises a picture extracting section that extracts the specific picture.

Preferably, the box producing apparatus further includes an inspection unit comprising: an image obtaining section that is provided downstream of the printing section, and obtains an image of the printed face which is printed on a box material sheet by the printing section; and a pass/fail determining section that determines whether a printing is pass or fail based on the image of the printed face obtained by the image obtaining section, wherein the image obtaining unit uses the image obtaining section in the inspection unit, rather than a dedicated image obtaining section.

Preferably, the register correction control unit obtains the master image from print plate data.

Preferably, the register correction control unit determines the amount of register displacement using a pattern matching method. In this case, as the pattern matching method, for example, a technique such as a residual matching, that a residual of overlap determines to be minimum point, may be used while moving the position of a master pattern (master image) horizontally and vertically with respect to a target pattern (the obtained image of the printed face).

Preferably, the image obtaining unit obtains an image of a printed face printed on the box material sheet which has been test-fed through the box producing apparatus, and the register correction control unit corrects the print register, based on the image of the printed face obtained by the image obtaining unit, as a pre-adjustment before a production operation of the box producing apparatus.

Preferably, the box producing apparatus further includes a slotter creaser section that is provided downstream of the printing section and comprises a slitter that slots the box material sheet; a slotting position detection apparatus that detects a position slotted by the slitter; and a slotting position controller that compares the slotting position detected by the slotting position detection apparatus and a sample slotting position to determine an amount of displacement of the slotting position, and corrects a slotting position by controlling an index position adjustment mechanism provided in the slitter, based on the amount of displacement of the slotting position.

Preferably, the box producing apparatus further includes a die cutting section that is provided downstream of the slotter creaser section and cuts the box material sheet; a cutting position detection apparatus that detects a cutting position cut by the die cutting section; and a cutting position controller that compares the cutting position detected by the cutting position detection apparatus and a sample cutting position to determine an amount of displacement of the cutting position, and corrects a cutting position by controlling an index position adjustment mechanism provided in the die cutting section, based on the amount of displacement of the cutting position.

Preferably, the box producing apparatus further includes a glue machine that is provided downstream of the die cutting section and glues the box material sheet; a gluing position detection apparatus that detects a gluing position glued by the glue machine; and a gluing position controller that compares the gluing position detected by the gluing position detection apparatus and a sample gluing position to determine an amount of displacement of the gluing position, and corrects a gluing position by controlling an index position adjustment mechanism provided in the glue machine, based on the amount of displacement of the gluing position.

Further, an inspection unit of the present invention is defined by claim 10 and is an inspection unit provided in a box producing apparatus comprising a printing section comprising a plurality of print units, the inspection unit comprising: an image obtaining section that obtains an image of a printed face which is printed on a box material sheet by the printing section; and an inspection section that inspects whether a printing is pass or fail based on the image of the printed face obtained by the image obtaining section; a register correction control section that compares a specific picture in a master image of a sample for the print, and a specific picture in the image of the printed face obtained by the image obtaining section to determine an amount of register displacement in each of the print units, and outputs register correction information based on the amount of register displacement to a register adjustment mechanism provided in each of the print units.

Preferably, the register correction control section relates the amount of register displacement to print plate data related to an ink color and outputs the amount of register displacement.

Further, a print register control method for a box producing apparatus of the present invention defined by claim 12 and is a method of adjusting a register for each of a plurality of print units in a box producing apparatus comprising a printing section comprising the print units, the method comprising: obtaining an image of a printed face which is printed on a box material sheet by the printing section; comparing a specific picture in a master image of a sample for the print, and the specific picture in the obtained image of the printed face to determine an amount of register displacement in each of the print units; and controlling a register adjustment mechanism provided in each of the print units to
correct a register of the print based on the amount of register displacement.

### Effect of the Invention

In accordance with the box producing apparatus, the inspection unit, and the print register control method of the present invention, an amount of register displacement for each of the print units is determined by comparing a picture in the specified region within the obtained image of the printed face against the specific picture in the master image of the sample for print. Then, based on the amount of register displacement, a register adjustment mechanism provided in each print unit is controlled to correct the register for print. For example, by obtaining master image information, identifying a picture to be printed by each print unit from that master image information, and specifying a region including that picture in advance, a print image can be obtained by the image obtaining unit by test feeding a single box material sheet. Then, a picture in the specified region in the print image is compared against the specific picture in the master image, and an amount of register displacement for a print plate of each of the print units is determined by the register correction control unit. Based on that amount of register displacement, a register adjustment mechanism in each of the print units is controlled to automatically correct the register for print. Accordingly, a pre-adjustment of the box producing apparatus can be facilitated, and the burden on an operator can be reduced. Without requiring the skill of the operator, boxes can be manufactured suitably swiftly in shorter time.

Further, since the image obtaining section including the image obtaining section and the pass/fail determining section in the inspection unit is used as an image obtaining unit, the same unit can be used for both the register control and the pass/fail inspection. Hence, the advantage in that the apparatus design can be simplified and the effect in that the cost for the apparatus can be reduced, can be obtained.

Additionally, since master images can be obtained from print plate data which is available upon the order, appropriate master images can be obtained easily and the preciseness of the control can be improved, while reducing the human burden.

Further, by specifying the specific picture as a picture including at least an area where a plurality of colors do not overlap, an amount of register displacement is determined without an error for a print unit in interest. By automatically extracting such a specific picture by the register correction control unit, the specific picture can be easily set.

The amount of register displacement is determined in a reliable and precise manner since a pattern matching method is used for the determination.

Further, in addition to correction of the register for print, the position slotted by the slitter is detected, an amount of displacement of the slotting position is determined by comparing the detected slotting position against the sample slotting position, and the slotting position is corrected based on the amount of displacement. This can improve the quality of box production.

Further, the cutting position cut by the die cutting section is detected, an amount of displacement of the cutting position is determined by comparing the detected cutting position against the sample cutting position, and the cutting position is corrected based on the amount of displacement. This can also improve the quality of box production.

Similarly, the gluing position glued by the glue machine is detected, an amount of displacement of the gluing position is determined by comparing the detected gluing position against the sample gluing position, and the gluing position is corrected based on the amount of displacement. This can also improve the quality of box production.

Note that, the determined amount of register displacement is output while relating it to print plate data related to an ink color, and hence, a register for print can be corrected by controlling the register adjustment mechanism in the print unit related to that ink color, even when the combinations of print plates and print units having the print plate attached thereto are modified.

### BRIEF DESCRIPTION OF THE DRAWINGS

FIG. 1 is a configuration diagram showing the configuration of a box producing apparatus and an inspection unit provided therein in accordance with a first embodiment of the present invention, together with the steps to process sheets;
FIG. 2 is a block diagram showing an information processing system in the box producing apparatus in accordance with the first embodiment of the present invention;
FIG. 3 is a configuration diagram showing a printing unit in a printing section in the box producing apparatus in accordance with the first embodiment of the present invention;
FIG. 4 is a perspective view of a main part of the print unit, illustrating the function of a register adjustment mechanism in the print unit in the printing section in the box producing apparatus in accordance with the first embodiment of the present invention;
FIG. 5 is a schematic diagram illustrating how a master image of a print sample is obtained and used in accordance with the first embodiment of the present invention;
FIG. 6 is a schematic diagram illustrating how an image of a printed face is obtained and processed in accordance with the first embodiment of the present invention;
FIG. 7 is a schematic diagram showing an example of a master image compared against an image of a printed face (sensor image) in accordance with the first embodiment of the present invention, wherein FIG. 7 (a) shows a sensor image and FIG. 7 (b) shows a master image;
FIG. 8 is a schematic diagram showing an example of calculation of an amount of displacement of print position in accordance with the first embodiment of the present invention, wherein FIG. 8 (a) to FIG. 8 (d) show the respective print units, specifically FIG. 8 (a) shows a first print unit, FIG. 8 (b) shows a second print unit, FIG. 8 (c) shows a third print unit, and FIG. 8 (d) shows a fourth print unit;
FIG. 9 is a flowchart illustrating control of print resister correction in accordance with the first embodiment of the present invention; and
FIG. 10 is a block diagram showing an information processing system in the box producing apparatus according to a second embodiment of the present invention.

### DESCRIPTION OF EMBODIMENTS

Hereinafter, embodiments of the prevent invention will be described with reference to the drawings.

### [FIRST EMBODIMENT]

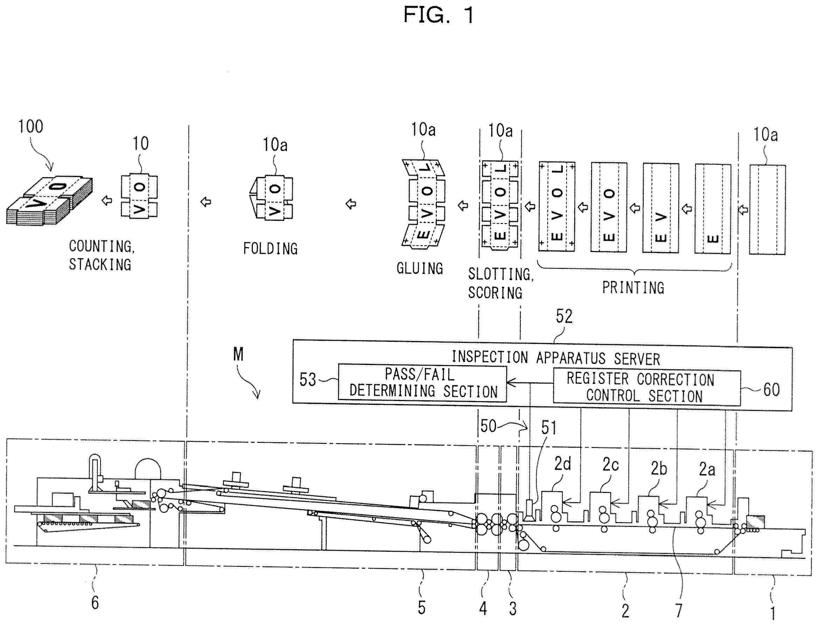

FIGS. 1 to 9 are diagrams illustrating a box producing apparatus and an inspection unit provided in the box producing apparatus, and a register correction method therefor, in accordance with a first embodiment of the present invention. Description will be given with reference to the drawings.

### (BOX PRODUCING APPARATUS)

Firstly, a box producing apparatus in accordance with the present embodiment will be described. FIG. 1 is a configuration diagram illustrating a box producing apparatus in accordance with the present embodiment. In FIG. 1, the apparatus configuration is illustrated in the bottom portion of the drawing, whereas the steps wherein a corrugated board sheet as box material sheet is processed into a flatly folded box (box producing sheet) is illustrated in the upper portion of the drawing, separately from the apparatus configuration.

As shown in FIG. 1, a box producing apparatus M is provided with, from the upstream side to the downstream side, a feed section 1, a printing section 2, a slotter creaser section 3, a die cutting section 4, a folding section (folder gluer section) 5, and a counter ejector section 6.

The feed section 1 is introduced, having a large number of plate-shaped corrugated board sheets 10a loaded thereon, and the corrugated board sheets 10a are supplied to the printing section 2 on a sheet by sheet basis on a transfer conveyer 7.

The printing section 2 includes print units 2a-2d, each supplied for one of a certain number of print colors (four colors in this example), and in the printing section 2, each print color ink is sequentially printed to the corrugated board sheets 10a supplied on a sheet by sheet basis by the transfer conveyer 7. In the example shown in FIG. 1, a picture "E" is printed by a first print unit 2a, a picture "V" is printed by a second print unit 2b, a picture "O" is printed by a third print unit 2c, and a picture "L" is printed by a fourth print unit 2d. The printing section will be described later in detail.

The slotter creaser section 3 includes a slitter and a scorer, and the corrugated board sheets 10a that have been printed by the printing section 2 undergo slotting by the slitter and scoring by the scorer.

The die cutting section 4 cuts and removes unnecessary sections from the corrugated board sheets 10a by die cutting.

A glue gun is provided in the most upstream of the folding section 5. Glue is applied by the glue gun to one of transverse gluing edges of the corrugated board sheets 10a that have been printed, slotted, scored, and die-cut. The corrugated board sheet 10a are then folded such that the transverse gluing edges of the corrugated board sheets 10a overlap on the rear side (lower side), and the transverse gluing edges of the corrugated board sheets 10a are bonded together with the glue to produce box producing sheets 10.

In the counter ejector section 6, the box producing sheets 10 processed in the folding section 5 are stacked on a stacker, while being counted. Once a certain number of box producing sheets 10 are stacked, the resultant sheet group 100 is transferred to a subsequent step.

### (INSPECTION UNIT)

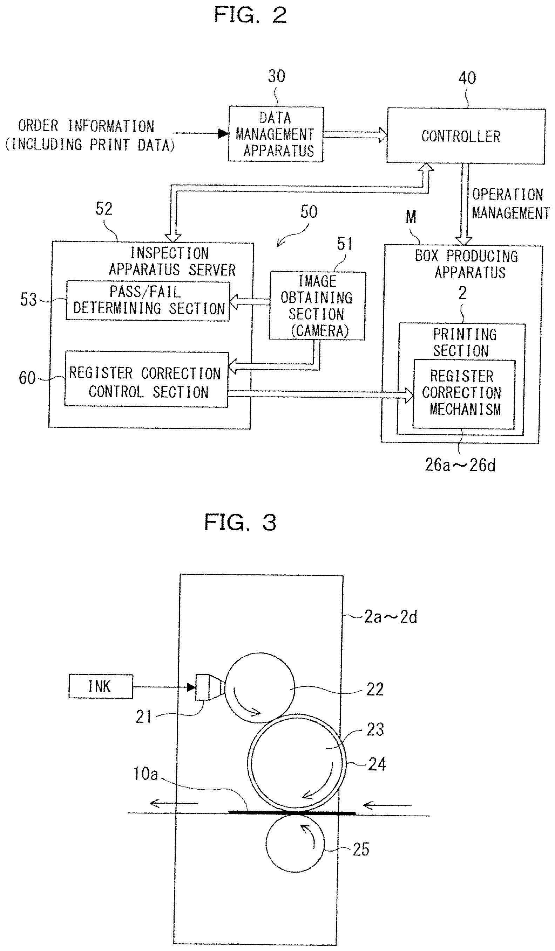

As shown in FIGS. 1 and 2, an inspection unit 50 in accordance with the present embodiment includes: a camera 51, as an image obtaining section (image obtaining unit) that obtains an image of a printed face which is printed on a corrugated board sheet during a normal operation of the box producing apparatus; a pass/fail determining section 53 that determines whether a printing is pass or fail by the printing section (whether print positions are pass or fail, or whether any color is missing or not) by comparing the image of the printed face obtained by camera 51, and a master image (reference image) of a sample (reference) for the print; and a register correction control section (resister correction control apparatus) 60 that compares the image of the printed face obtained by the camera 51, and the master image (reference image) of the sample (reference) for the print to determine an amount of displacement of the position (amount of register displacement) of the picture printed by each of the print units 2a-2d and corrects the register (adjusts the register) in each of the print units 2a-2d based on the amount of register displacement.

Note that the pass/fail determining section 53 and the register correction control section 60 are configured as function elements in an inspection unit server (server computer) 52 in the present embodiment. However, the inspection unit 50 may be configured to have the camera 51 and the inspection unit server 52 including the pass/fail determining section 53, while the register correction control section 60 may be configured from hardware (a computer for register correction control) separately from the inspection unit server 52, when communicatively connected to the inspection unit server 52.

The pass/fail determining section 53 only determines whether or not a picture corresponding to the position corresponding to each picture in the master image is present in the image of the printed face, during a normal operation of the box producing apparatus. In contrast, the register correction control section 60 determines, during a preparation stage before the normal operation (during pre-adjustment), an amount of displacement of a print picture in the image of the printed face corresponding to the picture of each ink color in the master image according to a picture of the sample of the print picture, to calculate an amount to be corrected for correcting a register in the print plate, and correct the register in each of the print units 2a-2d.

Hereinafter, the configurations of the print units 2a-2d will be described briefly. As shown in FIG. 3, each of the print units 2a-2d includes a flexo print machine, and includes an ink chamber 21 including a chamber frame, a seal blade, and a doctor blade; an anilox roll 22 which rotates while sliding together with the seal blade and the doctor blade, and receives flexo ink in grooves in the outer periphery face from the ink chamber 21; a printing cylinder 23 having a print plate 24 attached on the outer periphery thereof; the print plate 24 having the flexo ink transferred from the anilox roll 22; and an impression cylinder 25 pressing a corrugated board sheet 10a together with the printing cylinder 23.

Upon printing using a box producing apparatus, unlike typical printing machines for offset print where print units to be used depend on ink colors, inks to be used are all special colors and inks are assigned to print units 2a-2d differently depending on the case. Accordingly, when ink colors are assigned to the print units 2a-2d, the ink colors are associated with the print units 2a-2d and the associations of ink colors with print pictures are maintained in print plate data.

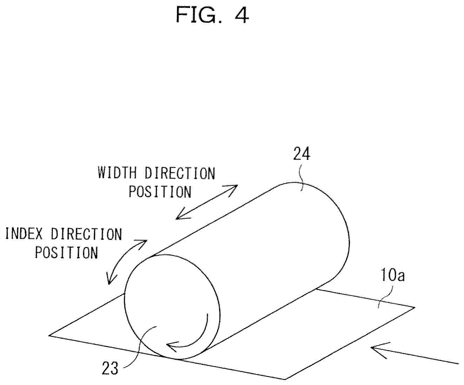

As shown in FIG. 4, the printing cylinder 23 includes register adjustment mechanisms 26a-26d (refer to FIG. 2) which can adjust the position in the index direction (position in the vertical direction) and the position in the width direction of the print plate 24. Each register adjustment mechanism operates in response to a control signal to adjust a register (correct the register) by adjusting the position in the index direction (position in the vertical direction) and the position in the width direction of the print plate 24.

Next, the register correction control will be described.

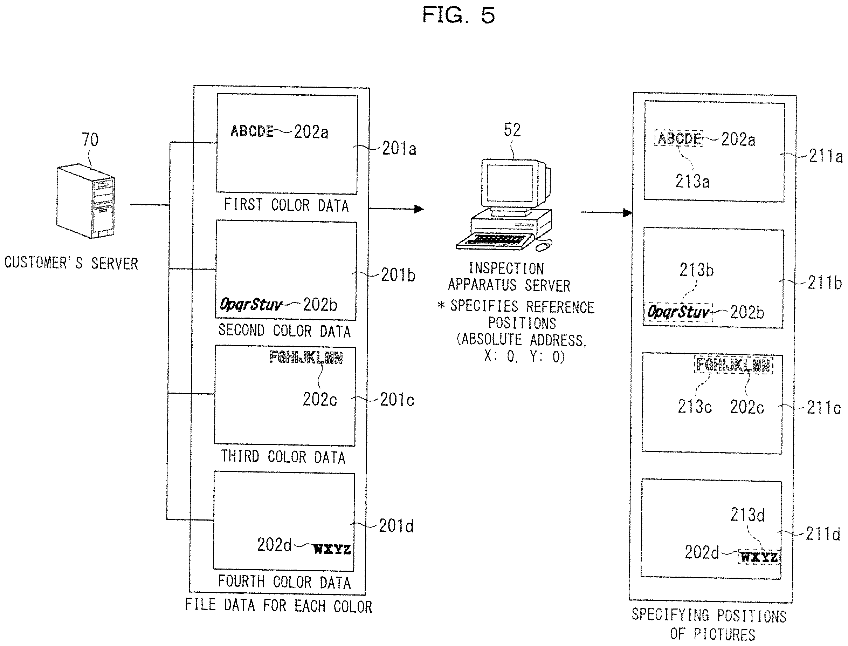

The register correction control requires data of a master image of a sample for printing, and as shown in FIG. 5, a box producing ordering server (customer's server) 70 sends data of a master image of a sample for printing to the inspection unit server 52. The master image data includes file data for each color (data of a print plate 24 for each print unit), and the register correction control section 60 in the inspection unit server 52 extracts a typical picture 202 for each color from file data 201 of that color, and specifies a region 213 including the extracted typical picture 202 in search window data 211. The register correction control section 60 in the inspection unit server 52 selects the region 213 which is sufficiently wide to include the typical picture 202 and where any other ink color is not present, as the position relative to the reference position (absolute address X0 and Y0). The typical picture 202 should satisfy these requirements.

Note that, that typical picture 202 is a picture which includes at least a region where multiple colors do not overlap, and is a picture where any other color is not present in the vicinity in the present embodiment. A picture which includes at least a region where multiple colors do not overlap, or a picture where any other color is not present in the vicinity, can be automatically extracted from plate making data as a region where multiple colors do not overlap or multiple colors are separated from each other. Although the register correction control section 60 extracts a typical picture 202 automatically (this function is referred to as a picture extracting section), the typical picture 202 may be selected manually, as a matter of course.

Although the region 213 including such a typical picture is automatically specified by the register correction control section 60 (this function is referred to as a region specifying section), the region 213 may also be manually set.

In the example shown, the "ABCDE" where no other color ink is present in the vicinity is extracted from print data (first color data) 201a by a first print unit as a typical picture 202a, and a region 213a including this typical picture 202a with sufficient margins is selected and specified in search window data 211a. Similarly, the "OpqrStuv" where no other color ink is present in the vicinity is extracted from print data (second color data) 201b by a second print unit as a typical picture 202b, and a region 213b including this typical picture 202b with sufficient margins is selected and specified in search window data 211b. Similarly, the "FGIJKLMN" where no other color ink is present in the vicinity is extracted from print data (third color data) 201c by a third print unit as a typical picture 202c, and a region 213c including this typical picture 202c with sufficient margins is selected and specified in search window data 211c. Similarly, the "WXYZ" where no other color ink is present in the vicinity is extracted from print data (fourth color data) 201d by a fourth print unit as a typical picture 202d, and a region 213d including this typical picture 202d with sufficient margins is selected and specified in search window data 211d.

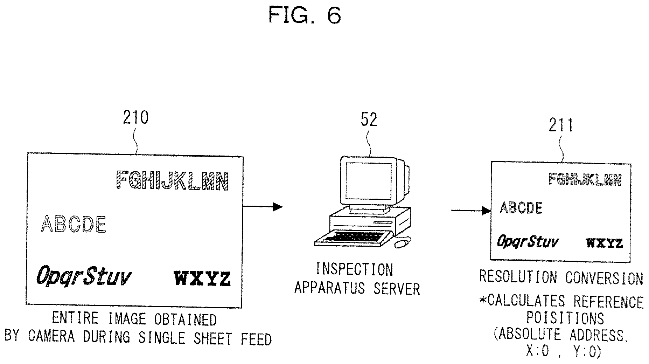

In contrast, the register correction control requires data of the image of the printed face (sensor image), which is printed using the print plate 24 based on print data, and the inspection unit server 52 obtains image data from the camera 51, as shown in FIG. 6. The image data is required to be obtained by feeding only a single corrugated board sheet 10a to the box producing apparatus M in a single sheet feed, provided as a test feed mode in the box producing apparatus M and then capturing the entire image of the corrugated board sheet 10a including all pictures printed on the printed face of the corrugated board sheet 10a, or be data of a region including all typical pictures 202a-202d for the respective ink colors and a reference position of the corrugated board sheet 10a (e.g., any of the corners). The camera 51 obtains an image that satisfies these requirements.

The register correction control section 60 in the inspection unit server 52 performs a resolution conversion to the resolution of the captured and recorded master image data, and specifies a reference position (absolute address X0 and Y0), as in search window data 211.

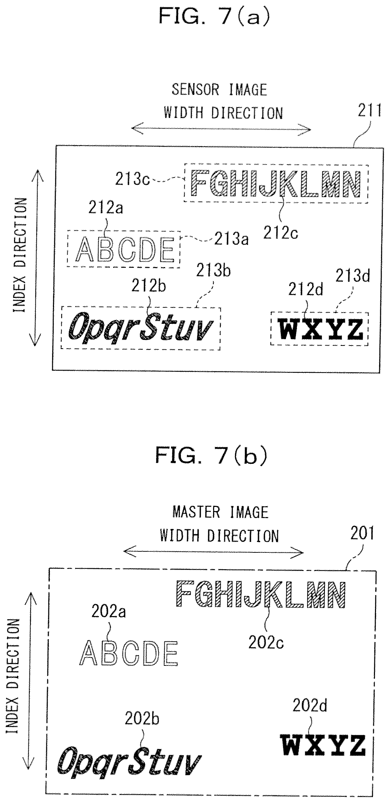

Once data (typical picture) 202 (202a-202d) in file data 201 (201a-201d) of the master image, and data (pictures corresponding to typical pictures) 212 (212a-212d) in search window data 211 (211a-211d) in the sensor image (the image of the printed face) are obtained in this manner, as shown in FIG. 7 (a) and FIG. 7 (b), the register correction control section 60 in the inspection unit server 52 searches for a picture 212 (212a-212d) in the region (window) 213 (213a-213d) specified for each color and matches the picture 212 (212a-212d) in the specified region 213 for each color with a corresponding picture 202 (202a-202d) in the master image, using the pattern matching method. In the manner as described above, upon matching a picture, an amount of positional displacement with respect to the master image of the image of the printed face is calculated from the corresponding positional information of that picture.

There are a wide variety of known pattern matching methods to be applied, including, for example, a residual matching that a residual of overlap determines to be a minimum point while moving the position of a master pattern horizontally and vertically with respect to a target pattern, a normalization correlation method, a phase limiting correlation, a geometrical matching and the like.

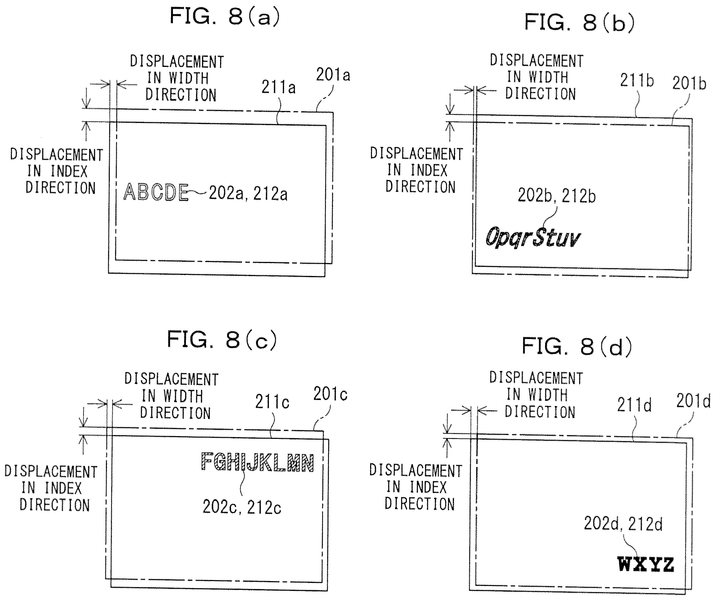

That is, as shown in FIG. 8 (a), for the ink color of the first print unit, when a typical picture 202a in the master image and a picture (corresponding picture) 212a corresponding to a typical picture in the sensor image are matched using the pattern matching method, a deviation arises in the reference positions (absolute addresses X0 and Y0) in the index direction and in the width direction. That deviation is an index direction displacement and a width direction displacement. Similarly, as shown in FIG. 8 (b) to FIG. 8 (d), for the ink colors of the second, third, and fourth print units, when typical pictures 202b, 202c, and 202d in the master image and pictures (corresponding pictures) 212b, 212c, and 212d corresponding to typical pictures in the sensor image are matched using the pattern matching method, respective deviations (i.e., index direction displacements and width direction displacements) arise in the reference positions (absolute addresses X0 and Y0) of the picture of the master image and the picture of the sensor image in the index direction and in the width direction.

In this manner, the register correction control section 60 in the inspection unit server 52 determines an index direction displacement and a width direction displacement (correctively referred to as "register displacement") for each print unit, and generates and outputs a control signal corresponding to a control amount for canceling the displacement for a register adjustment mechanism provided in the printing cylinder 23 for each print unit, corresponding to the index direction displacement and the width direction displacement. Note that the relationship of an adjustment amount (control amount) of the position in the index direction of the print plate 24 to the index direction displacement, and the relationship of an adjustment amount (control amount) of the position in the width direction of the print plate 24 to the width direction displacement are stored in memory in the inspection unit server 52 or an external memory, and is refereed to upon generation of a control signal.

Note that this register correction control section 60 outputs the determined amount of register displacement while relating it to print plate data related to an ink color, and hence, a register for print can be corrected by controlling the register adjustment mechanism in the print unit related to that ink color, even when the combination of print plates and print units having the print plate attached thereto is modified.

### (EFFECTS AND ADVANTAGES)

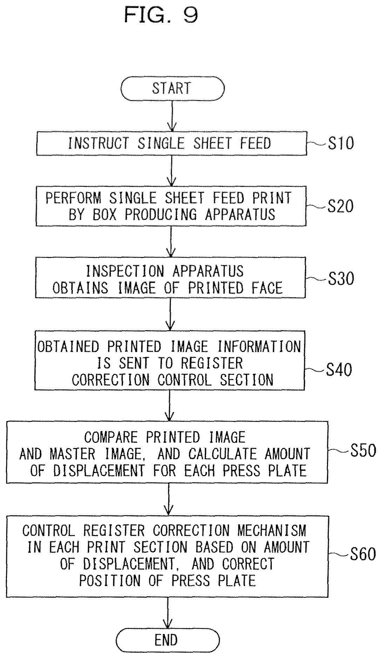

Since the box producing apparatus and the inspection unit in accordance with the first embodiment of the present invention are configured as described above, as shown in FIG. 9 for example, a register correction for a plate position can be performed as a pre-adjustment of the box producing apparatus.

More specifically, firstly, before the normal operation preparation stage (during the pre-adjustment), a single sheet feed is commanded through a button operation of the box producing apparatus M, and only a single corrugated board sheet is test-fed (Step S10). In response, the box producing apparatus M performs print on the single corrugated board sheet 10a that is fed (Step S20). Note that the box producing apparatus M processes up to folding and edge bonding after the printing.

Once the print is performed on the corrugated board sheet 10a, the camera 51 in the inspection unit 50 captures the printed face (Step S30) and information of the captured print image is sent to the register correction control unit (register correction control section) 60 (Step S40).

The register correction control unit (register correction control section) 60 compares the print image (sensor image) and the master image, calculates the amount of register displacement (index direction amount of displacement and width direction amount of displacement) for each plate (Step S50), and correct the plate position by controlling the register correction mechanism in each print unit in the printing section, based on the calculated amount of register displacement (Step S60).

Note that, upon determining the amount of register displacement, instead of paying attention to ink colors, each of the print units 2a-2d and the specific picture (typical picture) 202 printed by the print unit are related to each other, and a region 213 where the other ink colors are absent is selected and specified such that there is a sufficient margin with respect to the typical picture 202. Since the specific picture is searched for within that region 213, the amount of register displacement is determined by suitably extracting a picture related to each of the print units 2a-2d from the data of the sensor picture.

Accordingly, in accordance with the present box producing apparatus, the present inspection unit, and the present print register control method, an amount of register displacement for each of the print units 2a-2d is determined by comparing a picture in the specified region within the obtained image of the printed face against the specific picture in the master image of the sample for print. Then, based on the amount of register displacement, a register adjustment mechanism provided in each of the print units 2a-2d is controlled to correct the register for print. For example, by obtaining master image information, identifying a picture to be printed by each print unit from that master image information, and specifying a region including that picture in advance, a print image can be obtained by the camera 51 by test feeding a single box material sheet. Then, a picture in a specified region in the print image is compared against the specific picture in a master image, and an amount of register displacement for a print plate of each of the print units 2a-2d is determined by the register correction control section 60. Based on that amount of register displacement, a register adjustment mechanism in each of the print units 2a-2d is controlled to automatically correct the resister in the print plate. Accordingly, a pre-adjustment of the box producing apparatus can be facilitated, and the burden on an operator can be reduced. Without requiring the skill of the operator, boxes can be manufactured suitably in shorter time.

Further, since the camera 51 in the inspection unit 50 including the camera (image obtaining section) 51 and the pass/fail determining section 53 is used as an image obtaining unit that supplies image information to a register correction control section (register correction control unit) 60, the same unit can be used for both the register control and the pass/fail inspection. Hence, the advantage in that the apparatus design can be simplified and the effect in that the cost for the apparatus can be reduced, can be obtained.

Additionally, since master images can be obtained from print plate data which is available upon the order, appropriate master images can be obtained easily and the preciseness of the control can be improved, while reducing the burden on the operator.

The amount of register displacement is determined in a reliable and precise manner since a pattern matching method is used for the determination.

### [SECOND EMBODIMENT]

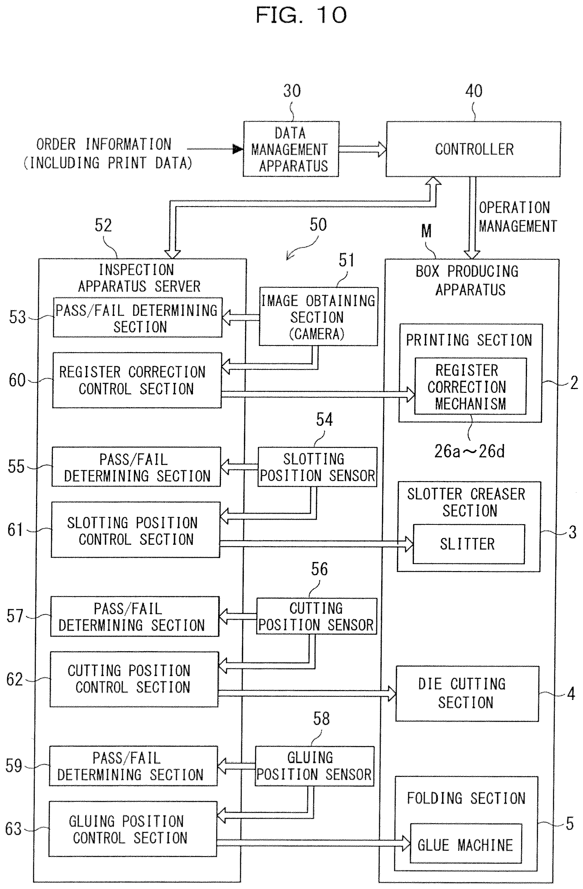

Next, a second embodiment of the present invention will be explained with reference to FIG. 10.

As shown in FIG. 10, the present embodiment employs an inspection unit that determines whether a produced box is pass or fail, similarly to the first embodiment. However, in addition to a camera 51 and a pass/fail determining section 53 that determines whether a printing by the printing section 2 is pass or fail (whether print positions are pass or fail, or whether any color is missing or not), the inspection unit in accordance with the present embodiment includes a sensor (slotting position detection apparatus) 54 that detects a position slotted by a slitter, a pass/fail determining section 55 that determines whether the slotting position is pass or fail by comparing the detected slotting position against a sample slotting position; a sensor (cutting position detection apparatus) 56 that detects a cutting position cut by a die cutting section; a pass/fail determining section 57 that determines whether the cutting position is pass or fail by comparing the detected cutting position against a sample cutting position; a sensor (gluing position detection apparatus) 58 that detects a gluing position glued by a glue machine; and a pass/fail determining section 59 that determines whether the gluing position is pass or fail by comparing the detected gluing position against a sample gluing position.

Additionally, in addition to a register correction control section (register correction control unit) 60, the present embodiment is provided with a slotting position control section (slotting position controller) 61 that determines an amount of displacement of the slotting position by comparing the slotting position detected by the slotting position sensor 54 with the sample slotting position, and controls an index position adjustment mechanism (not shown) provided in the slitter in the slotter creaser section 3 based on the amount of displacement of the slotting position to correct the slotting position; a cutting position control section (cutting position controller) 62 that determines an amount of displacement of the cutting position by comparing the cutting position detected by the cutting position sensor 56 with the sample cutting position, and controls an index position adjustment mechanism (not shown) provided in the die cutting section 4 based on the amount of displacement of the cutting position to correct the cutting position; and a gluing position control section (gluing position controller) 63 that determines an amount of displacement of the gluing position by comparing the gluing position detected by the gluing position sensor 58 with the sample gluing position, and controls an index position adjustment mechanism (not shown) provided in the glue machine in the folding section 5 based on the amount of displacement of the gluing position to correct the gluing position.

Note that a camera may be employed for each of the sensors 54, 56, and 58, for example. In this case, the camera 51 is positioned such that the camera 51 can be used as the sensors 54, 56, and 58 immediately after the glue machine, which can reduce the cost and simplify the apparatus design.

In the case of the present embodiment, in addition to the correction of the register for print, the slotted position slotted by the slitter is detected, an amount of displacement of the slotting position is determined by comparing the detected slotting position against the sample slotting position, and the slotting position is corrected based on the amount of displacement. This can improve the quality of box production.

Further, the cutting position cut by the die cutting section 4 is detected, an amount of displacement of the cutting position is determined by comparing the detected cutting position against the sample cutting position, and the cutting position is corrected based on the amount of displacement. This can also improve the quality of box production.

Similarly, the gluing position glued by the glue machine is detected, an amount of displacement of the gluing position is determined by comparing the detected gluing position against the sample gluing position, and the gluing position is corrected based on the amount of displacement. This can also improve the quality of box production.

### [MISCELLANEOUS]

Although embodiments of the present invention have been described, the present invention is not limited to the embodiments described above and various modifications may be made without departing from the spirit of the present invention.

For example, although inspection units also function as the units that correct displacements in the elements to reduce the cost and simplify the apparatus design in the above embodiment, separate sensors (including cameras) 51, 54, 56, and 58 and separate control sections (controllers) 53, 55, 57, and 59 may be provided.

Further, while print color information are obtained from related print plate data for printing (print color information are obtained together with the print plate data) in the above embodiment, information on the print color may be manually supplied (e.g., may be entered by a human by means of entry buttons) in the absence of print color information.

### INDUSTRIAL APPLICABILITY

The present invention can be applicable to a box producing apparatus that prints multiple colors on sheet faces and produces boxes, the box producing apparatus being provided with units to correct registers (adjust registers) for the print colors.

### Description of Reference Characters

- 1: Feed section
- 2: Printing section
- 2a-2d: Print unit
- 3: Slotter creaser section
- 4: Die cutting section
- 5: Folder gluer section
- 6: Counter ejector section
- 7: Transfer conveyer
- 10: Box producing sheet
- 10a: Corrugated board sheet (box material sheet)
- 21: Ink chamber
- 22: Anilox roll
- 23: Printing cylinder
- 24: Print plate
- 25: Impression cylinder
- 26a-26d: Register adjustment mechanism
- 50: Inspection unit
- 51: Camera (image obtaining unit)
- 52: Inspection unit server (server computer)
- 53, 55, 57, 59: Pass/fail determining section
- 54: Slotting position sensor (slotting position detection apparatus)
- 56: Cutting position sensor (cutting position detection apparatus)
- 58: Gluing position sensor (gluing position detection apparatus)
- 60: Register correction control section (register correction control unit)
- 61: Slotting position control section (slotting position controller)
- 62: Cutting position control section (cutting position controller)
- 63: Gluing position control section (gluing position controller)
- 70: Box producing ordering server (customer's server)
- 100: Box producing sheet group

## Claims

1. A box producing apparatus comprising:
a printing section (2) comprising a plurality of print units (2a-2d) including a flexo print machine;
an image obtaining unit (51) configured to obtain an image of a printed face which is printed on a box material sheet (10a) by the printing section (2); and
a register correction control unit (60) configured to compare a typical picture (202) in a master image of a sample for the print, and a corresponding pictures (212) corresponding to the typical picture in the image of the printed face obtained by the image obtaining unit (51) to determine an amount of register displacement in each of the print units (2a-2d), and to control a register adjustment mechanism (26a-26d) provided in each of the print units (2a-2d) based on the amount of register displacement to correct a register of the print, wherein
the typical picture (202) is a picture including at least an area where a plurality of colors do not overlap, and
the register correction control unit (60) comprises a picture extracting section that extracts the typical picture (202).

2. The box producing apparatus according to claim 1, wherein the register correction control unit (60) is configured to detect the corresponding picture (212) in a region of the printed face where the typical picture (202) is specified to exist in advance based on the master image, and to compare the corresponding picture (212) detected in the printed image against the typical picture (202) in the master image.

3. The box producing apparatus according to claim 1 or 2, further comprising:
an inspection unit (50) comprising:
an image obtaining section that is provided downstream of the printing section (2), and configured to obtain an image of the printed face which is printed on a box material sheet (10a) by the printing section (2); and
a pass/fail determining section (53,55,57,59) configured to determine whether a printing is pass or fail based on the image of the printed face obtained by the image obtaining section,
wherein the image obtaining unit (51) is configured to use the image obtaining section in the inspection unit (50), rather than a dedicated image obtaining section.

4. The box producing apparatus according to any one of claims 1 to 3, wherein the register correction control unit (60) is configured to obtain the master image from print plate data.

5. The box producing apparatus according to any one of claims 1 to 4, wherein the register correction control unit (60) is configured to determine the amount of register displacement using a pattern matching method.

6. The box producing apparatus according to any one of claims 1 to 5, wherein
the image obtaining unit (51) is configured to obtain an image of a printed face printed on the box material sheet (10a) which has been test-fed through the box producing apparatus, and
the register correction control unit (60) is configured to correct the print register, based on the image of the printed face obtained by the image obtaining unit (51), as a pre-adjustment before a production operation of the box producing apparatus.

7. The box producing apparatus according to any one of claims 1 to 6, further comprising:
a slotter creaser section (3) that is provided downstream of the printing section (2) and comprises a slitter configured to slots the box material sheet (10a);
a slotting position detection apparatus (54) configured to detect a position slotted by the slitter; and
a slotting position controller (61) configured to compare the slotting position detected by the slotting position detection apparatus (54) and a sample slotting position to determine an amount of displacement of the slotting position, and to correct a slotting position by controlling an index position adjustment mechanism provided in the slitter, based on the amount of displacement of the slotting position.

8. The box producing apparatus according to any one of claims 1 to 7, further comprising:
a die cutting section (4) that is provided downstream of a slotter creaser section (3) and configured to cut the box material sheet (10a);
a cutting position detection apparatus (56) configured to detect a cutting position cut by the die cutting section (4); and
a cutting position controller (62) configured to compare the cutting position detected by the cutting position detection apparatus (56) and a sample cutting position to determine an amount of displacement of the cutting position, and to correct a cutting position by controlling an index position adjustment mechanism provided in the die cutting section (4), based on the amount of displacement of the cutting position.

9. The box producing apparatus according to any one of claims 1 to 8, further comprising:
a glue machine that is provided downstream of a die cutting section (4) and configured to glue the box material sheet (10a);
a gluing position detection apparatus (58) configured to detect a gluing position glued by the glue machine; and
a gluing position controller (63) configured to compare the gluing position detected by the gluing position detection apparatus (58) and a sample gluing position to determine an amount of displacement of the gluing position, and to correct a gluing position by controlling an index position adjustment mechanism provided in the glue machine, based on the amount of displacement of the gluing position.

10. An inspection unit provided in a box producing apparatus comprising a printing section (2) comprising a plurality of print units (2a-2d) including a flexo print machine, the inspection unit comprising:
an image obtaining section (51) configured to obtain an image of a printed face which is printed on a box material sheet (10a) by the printing section (2);
an inspection section configured to inspect whether a printing is pass or fail based on the image of the printed face obtained by the image obtaining section (51), and
a register correction control section (60) configured to compare a typical picture (202) in a master image of a sample for the print, and a corresponding picture (212) corresponding to the typical picture (202) in the image of the printed face obtained by the image obtaining section (51) to determine an amount of register displacement in each of the print units (2a-2d), and to output register correction information based on the amount of register displacement to a register adjustment mechanism (26a-26d) provided in each of the print units (2a-2d), wherein
the typical picture (202) is a picture including at least an area where a plurality of colors do not overlap, and
the register correction control unit (60) comprises a picture extracting section that extracts the typical picture (202).

11. The inspection unit according to claim 10, wherein the register correction control section (60) is configured to relate the amount of register displacement to print plate data related to an ink color and to output the amount of register displacement.

12. A method of adjusting a register for each of a plurality of print units (2a-2d) in a box producing apparatus comprising a printing section (2) comprising the print units (2a-2d) including a flexo print machine, the method comprising:
obtaining an image of a printed face which is printed on a box material sheet (10a) by the printing section (2);
extracting a typical picture (202) which is a picture including at least an area where a plurality of colors do not overlap in a master image of a sample print,
comparing the extracted typical picture (202), and a corresponding picture (212) corresponding to the typical picture (202) in the obtained image of the printed face to determine an amount of register displacement in each of the print units (2a-2d); and
controlling a register adjustment mechanism (26a-26d) provided in each of the print units (2a-2d) to correct a register of the print based on the amount of register displacement.

## Patentansprüche

1. Schachtelherstellungsvorrichtung, umfassend:
eine Drucksektion (2), umfassend mehrere Druckeinheiten (2a-2d) einschließlich einer Flexodruckmaschine,
eine Bilderlangungseinheit (51), die dazu ausgelegt ist, ein Bild einer bedruckten Fläche zu erlangen, die von der Drucksektion (2) auf einen Schachtelmaterialbogen (10a) gedruckt ist, und
eine Registerkorrektur-Steuereinheit (60), die zum Vergleichen einer typischen Abbildung (202) in einer Bildvorlage eines Musters für den Druck und einer entsprechenden Abbildung (212), die der typischen Abbildung in dem von der Bilderlangungseinheit (51) erlangten Bild der bedruckten Fläche entspricht, um ein Ausmaß an Registerverschiebung in jeder der Druckeinheiten (2a-2d) zu bestimmen, und zum Steuern eines Registerjustiermechanismus (26a-26d), der in jeder der Druckeinheiten (2a-2d) bereitgestellt ist, auf der Grundlage des Umfangs an Registerverschiebung, um ein Register des Drucks zu korrigieren, ausgelegt ist, wobei
die typische Abbildung (202) eine Abbildung ist, die mindestens einen Bereich beinhaltet, in dem mehrere Farben nicht überlappen, und
die Registerkorrektur-Steuereinheit (60) eine Abbildungsextraktionssektion umfasst, die die typische Abbildung (202) extrahiert.

2. Schachtelherstellungsvorrichtung nach Anspruch 1, wobei die Registerkorrektur-Steuereinheit (60) zum Erkennen der entsprechenden Abbildung (212) in einer Region der bedruckten Fläche, in der die typische Abbildung (202) laut vorheriger Angabe auf der Grundlage der Bildvorlage existieren soll, und zum Vergleichen der in dem gedruckten Bild erkannten entsprechenden Abbildung (212) mit der typischen Abbildung (202) in der Bildvorlage ausgelegt ist.

3. Schachtelherstellungsvorrichtung nach Anspruch 1 oder 2, ferner umfassend:
eine Inspektionseinheit (50), umfassend:
eine Bildererlangungssektion, die der Drucksektion (2) nachgeordnet bereitgestellt und zum Erlangen eines Bildes der bedruckten Fläche, die von der Drucksektion (2) auf einen Schachtelmaterialbogen (10a) gedruckt ist, ausgelegt ist und
eine Akzeptabel-Nichtakzeptabel-Bestimmungssektion (53, 55, 57, 59), die dazu ausgelegt ist, auf der Grundlage des Bildes der bedruckten Fläche, das von der Bilderlangungssektion erlangt wurde, zu bestimmen, ob ein Druck akzeptabel oder nicht akzeptabel ist,
wobei die Bilderlangungseinheit (51) dazu ausgelegt ist, anstatt einer dedizierten Bilderlangungssektion die Bilderlangungssektion der Inspektionseinheit (50) zu benutzen.

4. Schachtelherstellungsvorrichtung nach einem der Ansprüche 1 bis 3, wobei die Registerkorrektur-Steuereinheit (60) dazu ausgelegt ist, die Bildvorlage aus den Druckplattendaten zu erlangen.

5. Schachtelherstellungsvorrichtung nach einem der Ansprüche 1 bis 4, wobei die Registerkorrektur-Steuereinheit (60) dazu ausgelegt ist, das Ausmaß der Registerverschiebung unter Verwendung eines Musterabgleichsverfahrens zu bestimmen.

6. Schachtelherstellungsvorrichtung nach einem der Ansprüche 1 bis 5, wobei
die Bilderlangungseinheit (51) dazu ausgelegt ist, ein Bild einer bedruckten Fläche, die auf den Schachtelmaterialbogen (10a) gedruckt ist, der zu Testzwecken durch die Schachtelherstellungsvorrichtung geführt wurde, zu erlangen und
die Registerkorrektur-Steuereinheit (60) dazu ausgelegt ist, das Druckregister auf der Grundlage des von der Bilderlangungseinheit (51) erlangten Bildes der bedruckten Fläche als Vorjustierung vor einem Herstellungsvorgang der Schachtelherstellungsvorrichtung zu korrigieren.

7. Schachtelherstellungsvorrichtung nach einem der Ansprüche 1 bis 6, ferner umfassend:
eine Schlitz-Falz-Sektion (3), die der Drucksektion (2) nachgeordnet bereitgestellt ist und einen Schlitzer umfasst, der dazu ausgelegt ist, den Schachtelmaterialbogen (10a) zu schlitzen,
eine Schlitzposition-Erkennungsvorrichtung (54), die dazu ausgelegt ist, eine von dem Schlitzer geschlitzte Position zu erkennen, und
eine Schlitzposition-Steuerung (61), die dazu ausgelegt ist, die von der Schlitzposition-Erkennungsvorrichtung (54) erkannte Schlitzposition und eine Muster-Schlitzposition zu vergleichen, um ein Ausmaß einer Verschiebung der Schlitzposition zu bestimmen und eine Schlitzposition durch Steuern eines in dem Schlitzer vorgesehenen Indexposition-Justiermechanismus auf der Grundlage des Ausmaßes der Verschiebung der Schlitzposition zu korrigieren.

8. Schachtelherstellungsvorrichtung nach einem der Ansprüche 1 bis 7, ferner umfassend:
eine Stanzsektion (4), die der Schlitz-Falz-Sektion (3) nachgeordnet vorgesehen ist und die dazu ausgelegt ist, den Schachtelmaterialbogen (10a) zu stanzen,
eine Stanzposition-Erkennungsvorrichtung (56), die dazu ausgelegt ist, eine von der Stanzsektion (4) gestanzte Stanzposition zu erkennen, und
eine Stanzposition-Steuerung (62), die dazu ausgelegt ist, die von der Stanzposition-Erkennungsvorrichtung (56) erkannte Stanzposition und eine Muster-Stanzposition zu vergleichen, um ein Ausmaß einer Verschiebung der Stanzposition zu bestimmen, und eine Stanzposition durch Steuern eines in der Stanzsektion (4) bereitgestellten Indexposition-Justiermechanismus auf der Grundlage des Ausmaßes der Verschiebung der Stanzposition zu korrigieren.

9. Schachtelherstellungsvorrichtung nach einem der Ansprüche 1 bis 8, ferner umfassend:
eine Klebemaschine, die der Stanzsektion (4) nachgeordnet vorgesehen ist und die dazu ausgelegt ist, den Schachtelmaterialbogen (10a) zu kleben,
eine Klebeposition-Erkennungsvorrichtung (58), die dazu ausgelegt ist, eine von der Klebemaschine geklebte Klebeposition zu erkennen, und
eine Klebeposition-Steuerung (63), die dazu ausgelegt ist, die von der Klebeposition-Erkennungsvorrichtung (58) erkannte Klebeposition und eine Muster-Klebeposition zu vergleichen, um ein Ausmaß einer Verschiebung der Klebeposition zu bestimmen, und eine Klebeposition durch Steuern eines in der Klebemaschine bereitgestellten Indexposition-Justiermechanismus auf der Grundlage des Ausmaßes der Verschiebung der Klebeposition zu korrigieren.

10. Inspektionseinheit, die in einer Schachtelherstellungsvorrichtung vorgesehen ist, umfassend eine Drucksektion (2), umfassend mehrere Druckeinheiten (2a-2d) einschließlich einer Flexodruckmaschine, wobei die Inspektionseinheit umfasst:
eine Bilderlangungssektion (51), die dazu ausgelegt ist, ein Bild einer bedruckten Fläche zu erlangen, die von der Drucksektion (2) auf einen Schachtelmaterialbogen (10a) gedruckt ist,
eine Inspektionssektion, die dazu ausgelegt ist, auf der Grundlage des Bildes der bedruckten Fläche, das von der Bilderlangungssektion (51) erlangt wurde, zu inspizieren, ob ein Druck akzeptabel oder nicht akzeptabel ist, und
eine Registerkorrektur-Steuersektion (60), die dazu ausgelegt ist, eine typische Abbildung (202) in einer Bildvorlage eines Musters für den Druck und einer entsprechenden Abbildung (212), die der typischen Abbildung (202) in dem von der Bilderlangungssektion (51) erlangten Bild der bedruckten Fläche entspricht, zu vergleichen um ein Ausmaß der Registerverschiebung in jeder der Druckeinheiten (2a-2d) zu bestimmen, und Registerkorrekturdaten auf der Grundlage des Ausmaßes der Registerverschiebung an einen Registerjustiermechanismus (26a-26d), der in jeder der Druckeinheiten (2a-2d) bereitgestellt ist, auszugeben, wobei
die typische Abbildung (202) eine Abbildung ist, die mindestens einen Bereich beinhaltet, in dem mehrere Farben nicht überlappen, und
die Registerkorrektur-Steuereinheit (60) eine Abbildungsextraktionssektion umfasst, die die typische Abbildung (202) extrahiert.

11. Inspektionseinheit nach Anspruch 10, wobei die Registerkorrektur-Steuersektion (60) dazu ausgelegt ist, das Ausmaß der Registerverschiebung mit Druckplattendaten, die auf eine Druckfarbe bezogen sind, in Beziehung zu setzen und das Ausmaß der Registerverschiebung auszugeben.

12. Verfahren zum Justieren eines Registers für jede von mehreren Druckeinheiten (2a-2d) in einer Schachtelherstellungsvorrichtung, umfassend eine Drucksektion (2), umfassend die Druckeinheiten (2a-2d) einschließlich einer Flexodruckmaschine, wobei das Verfahren umfasst:
Erlangen eines Bildes einer gedruckten Fläche, die von der Drucksektion (2) auf einen Schachtelmaterialbogen (10a) gedruckt ist,
Extrahieren einer typischen Abbildung (202), die eine Abbildung ist, welche mindestens einen Bereich in einer Bildvorlage eines Musters für den Druck beinhaltet, in dem mehrere Farben nicht überlappen,
Vergleichen der extrahierten Abbildung (202) und einer entsprechenden Abbildung (212), die der typischen Abbildung (202) in dem erlangten Bild der bedruckten Fläche entspricht, um ein Ausmaß der Registerverschiebung in jeder der Druckeinheiten (2a-2d) zu bestimmen, und
Steuern eines Registerjustiermechanismus (26a-26d), der in jeder der Druckeinheiten (2a-2d) vorgesehen ist, auf der Grundlage des Ausmaßes der Registerverschiebung, um ein Register des Drucks zu korrigieren.

## Revendications

1. Appareil de fabrication de boîtes, comprenant :
une section d'impression (2) comprenant une pluralité d'unités d'impression (2a à 2d) incluant une machine d'impression flexographique ;
une unité d'obtention d'image (51) configurée pour obtenir une image de face imprimée qui est imprimée sur une feuille de matériau de boîte (10a) par la section d'impression (2) ; et
une unité de commande de correction de repérage (60) configurée pour comparer une figure typique (202) dans une image maître d'un échantillon pour l'impression, et une figure correspondante (212) correspondant à la figure typique sur l'image de la face imprimée obtenue par l'unité d'obtention d'image (51) afin de déterminer une grandeur de décalage de repérage dans chacune des unités d'impression (2a à 2d), et pour commander un mécanisme d'ajustement de repérage (26a à 26d) se trouvant dans chacune des unités d'impression (2a à 2d) sur la base de la grandeur de décalage de repérage afin de corriger un repérage de l'impression, dans lequel
la figure typique (202) est une figure incluant au moins une zone où une pluralité de couleurs ne se chevauchent pas, et
l'unité de commande de correction de repérage (60) comprend une section d'extraction de figure qui extrait la figure typique (202).

2. Appareil de fabrication de boîtes selon la revendication 1, dans lequel l'unité de commande de correction de repérage (60) est configurée pour détecter la figure correspondante (212) dans une région de la face imprimée où il est spécifié que la figure typique (202) existe, à l'avance, sur la base de l'image maître, et pour comparer la figure correspondante (212) détectée sur l'image imprimée avec la figure typique (202) de l'image maître.

3. Appareil de fabrication de boîtes selon la revendication 1 ou 2, comprenant en outre :
une unité de contrôle (50) comprenant :
une section d'obtention d'image prévue en aval de la section d'impression (2), et qui est configurée pour obtenir une image de la face imprimée qui est imprimée sur une feuille de matériau de boîte (10a) par la section d'impression (2) ; et
une section de détermination de succès/échec (53, 55, 57, 59) configurée pour déterminer si une impression a réussi ou a échoué, sur la base de l'image de la face imprimée obtenue par la section d'obtention d'image,
dans lequel l'unité d'obtention d'image (51) est configurée pour utiliser la section d'obtention d'image de l'unité de contrôle (50), plutôt qu'une section dédiée d'obtention d'image.

4. Appareil de fabrication de boîtes selon l'une quelconque des revendications 1 à 3, dans lequel l'unité de commande de correction de repérage (60) est configurée pour obtenir l'image maître à partir de données de la plaque d'impression.

5. Appareil de fabrication de boîtes selon l'une quelconque des revendications 1 à 4, dans lequel l'unité de commande de correction de repérage (60) est configurée pour déterminer la grandeur de décalage de repérage à l'aide d'un procédé d'appariement de formes.

6. Appareil de fabrication de boîtes selon l'une quelconque des revendications 1 à 5, dans lequel :
l'unité d'obtention d'image (51) est configurée pour obtenir une image de face imprimée qui est imprimée sur la feuille de matériau de boîte (10a) qui a été introduite pour essai à travers l'appareil de fabrication de boîtes, et
l'unité de commande de correction de repérage (60) est configurée pour corriger le repérage d'impression, sur la base de l'image de la face imprimée obtenue par l'unité d'obtention d'image (51), en tant que préréglage avant une opération de fabrication de l'appareil de fabrication de boîtes.

7. Appareil de fabrication de boîtes selon l'une quelconque des revendications 1 à 6, comprenant en outre :
une section d'entaillage et de rainurage (3) prévue en aval de la section d'impression (2) et qui comprend un dispositif d'entaillage configuré pour entailler la feuille de matériau de boîte (10a) ;
un appareil de détection de position d'entaillage (54) configuré pour détecter une position entaillée par le dispositif d'entaillage ; et
un dispositif de commande de position d'entaillage (61) configuré pour comparer la position d'entaillage détectée par l'appareil de détection de position d'entaillage (54) et une position d'entaillage d'échantillon afin de déterminer une grandeur de décalage de la position d'entaillage, et de corriger une position d'entaillage en commandant un mécanisme d'ajustement de position d'indexage situé dans le dispositif d'entaillage, sur la base de la grandeur de décalage de la position d'entaillage.

8. Appareil de fabrication de boîtes selon l'une quelconque des revendications 1 à 7, comprenant en outre :
une section de découpage à l'emporte-pièce (4) prévue en aval d'une section d'entaillage et de rainurage (3) et qui est configurée pour découper la feuille de matériau de boite (10a) ;
un appareil de détection de position de coupe (56) configuré pour détecter une position de coupe découpée par le dispositif de découpage à l'emporte-pièce (4) ; et
un dispositif de commande de position de coupe (62) configuré pour comparer la position de coupe détectée par l'appareil de détection de position de coupe (56) et une position de coupe d'échantillon afin de déterminer une grandeur de décalage de la position de coupe, et de corriger une position de coupe en commandant un mécanisme d'ajustement de position d'indexage se trouvant dans le dispositif de découpage à l'emporte-pièce (4), sur la base de la grandeur de décalage de la position de coupe.

9. Appareil de fabrication de boîtes selon l'une quelconque des revendications 1 à 8, comprenant en outre :
une machine de collage prévue en aval d'une section de découpage à l'emporte-pièce (4) et qui est configurée pour coller la feuille de matériau de boîte (10a) ;
un appareil de détection de position de collage (58) configuré pour détecter une position de collage collée par la machine de collage ; et
un dispositif de commande de position de collage (63) configuré pour comparer la position de collage détectée par l'appareil de détection de position de collage (58) et une position de collage d'échantillon afin de déterminer une grandeur de décalage de la position de collage, et de corriger une position de collage en commandant un mécanisme d'ajustement de position d'indexage se trouvant dans la machine de collage, sur la base de la grandeur de décalage de la position de collage.

10. Unité de contrôle prévue dans un appareil de fabrication de boîtes comprenant une section d'impression (2) comprenant une pluralité d'unités d'impression (2a à 2d) incluant une machine d'impression flexographique, l'unité de contrôle comprenant :
une section d'obtention d'image (51) configurée pour obtenir une image de face imprimée qui est imprimée sur une feuille de matériau de boîte (10a) par la section d'impression (2) ;
une section de contrôle configurée pour contrôler si une impression a réussi ou a échoué, sur la base de l'image de la face imprimée obtenue par la section d'obtention d'image (51), et
une section de commande de correction de repérage (60) configurée pour comparer une figure typique (202) sur une image maître d'un échantillon pour l'impression, et une figure correspondante (212) correspondant à la figure typique (202) sur l'image de la face imprimée obtenue par la section d'obtention d'image (51) afin de déterminer une grandeur de décalage de repérage dans chacune des unités d'impression (2a à 2d), et pour délivrer des informations de correction de repérage, sur la base de la grandeur de décalage de repérage à un mécanisme d'ajustement de repérage (26a à 26d) se trouvant dans chacune des unités d'impression (2a à 2d), dans lequel :
la figure typique (202) est une figure incluant au moins une zone où une pluralité de couleurs ne se chevauchent pas, et
la unité de commande de correction de repérage (60) comprend une section d'extraction de figure qui extrait la figure typique (202).

11. Unité de contrôle selon la revendication 10, dans lequel la section de commande de correction de repérage (60) est configurée pour mettre en relation la grandeur de décalage de repérage avec des données de la plaque d'impression se rapportant à une couleur d'encre et pour délivrer la grandeur de décalage de repérage.

12. Procédé d'ajustement de repérage de chaque unité d'une pluralité d'unités d'impression (2a à 2d) dans un appareil de fabrication de boîtes comprenant une section d'impression (2) comprenant les unités d'impression (2a à 2d) incluant une machine d'impression flexographique, le procédé comprenant:
obtenir une image de face imprimée qui est imprimée sur une feuille de matériau de boîte (10a) par la section d'impression (2) ;
extraire une figure typique (202) qui est une figure incluant au moins une zone où une pluralité de couleurs ne se chevauchent pas sur une image maître d'impression d'échantillon,
comparer la figure typique extraite (202) et une figure correspondante (212) correspondant à la figure typique (202) sur l'image obtenue de la face imprimée afin de déterminer une grandeur de décalage de repérage dans chacune des unités d'impression (2a à 2d) ; et
commander un mécanisme d'ajustement de repérage (26a à 26d) prévu dans chacune des unités d'impression (2a à 2d) pour corriger un repérage de l'impression sur la base de la grandeur de décalage de repérage.
